## Europäisches Patentamt
## European Patent Office
## Office européen des brevets

(11) Veröffentlichungsnummer: **0 156 988**
**B1**

(12) # EUROPÄISCHE PATENTSCHRIFT

(45) Veröffentlichungstag der Patentschrift:
**22.07.87**

(51) Int. Cl.⁴: **G 03 B 42/02,** A 61 B 6/00

(21) Anmeldenummer: **84115566.6**

(22) Anmeldetag: **17.12.84**

(54) **Röntgendiagnostikgerät.**

(30) Priorität: **28.02.84 DE 3407221**

(43) Veröffentlichungstag der Anmeldung:
**09.10.85 Patentblatt 85/41**

(45) Bekanntmachung des Hinweises auf die Patenterteilung:
**22.07.87 Patentblatt 87/30**

(84) Benannte Vertragsstaaten:
**DE FR GB IT**

(56) Entgegenhaltungen:
**DE-C-831 802**
**FR-A-1 165 776**
**FR-A-2 142 377**

(73) Patentinhaber: **Siemens Aktiengesellschaft Berlin und München, Wittelsbacherplatz 2, D-8000 München 2 (DE)**

(72) Erfinder: **Saffer, Edmund, Pestalozzistrasse 4, D-8551 Eggolsheim (DE)**

LIBER STOCKHOLM 1987

## Beschreibung

Die Erfindung betrifft ein Röntgendiagnostikgerät für Röntgenaufnahmen mit einer Auflageplatte für das Aufnahmeobjekt, die einen Aufnahmeraum für eine Röntgenfilmkassette und ein diesen Aufnahmeraum überdeckendes Sekundärstrahlenraster aufweist.

Ein Röntgendiagnostikgerät dieser Art kann zur Anfertigung von Mammographieaufnahmen verwendet werden. In diesem Fall wird das Aufnahmeobjekt für die Anfertigung einer Röntgenaufnahme zwischen der Auflageplatte und einer Kompressionsvorrichtung gehalten. Das Sekundärstrahlenraster dämpft die vom Aufnahmeobjekt ausgehende Sekundärstrahlung. Allerdings vergrößert es den Abstand zwischen dem Aufnahmeobjekt und dem Röntgenfilm. In der Praxis besteht daher der Wunsch, auch Aufnahmen ohne Sekundärstrahlenraster anfertigen zu können.

Der Erfindung liegt demgemäß die Aufgabe zugrunde, ein Röntgendiagnostikgerät der eingangs genannten Art so auszubilden, daß wahlweise Aufnahmen mit und ohne Sekundärstrahlenraster anfertigbar sind.

Diese Aufgabe ist erfindungsgemäß dadurch gelöst, daß die Auflageplatte um eine senkrecht zum Zentralstrahl des Röntgenstrahlenbündels liegende Achse drehbar gelagert ist, derart, daß das Sekundärstrahlenraster wahlweise in Strahlenrichtung vor oder hinter der jeweils etngelegten Röntgenfilmkassette angeordnet werden kann. Bei dem erfindungsgemäßen Röntgendiagnostikgerät können Röntgenaufnahmen ohne Sekundärstrahlenraster in einfacher Weise durch Verdrehung der Auflageplatte angefertigt werden. Das mechanisch empfindliche Sekundärstrahlenraster muß hierzu nicht aus der Auflageplatte entfernt werden.

Eine zweckmäßige Weiterbildung der Erfindung besteht darin, daß auf der dem Sekundärstrahlenraster abgewandten Seite der Auflageplatte ein Fach für die Aufnahme einer Röntgenfilmkassette angeordnet ist. Bei dieser Weiterbildung kann auf der dem Sekundärstrahlenraster abgewandten Seite der Aufnahmeplatte der Strahlendetektor eines Belichtungsautomaten angeordnet sein, der demgemäß die Strahlendosis nach dem Röntgenfilm erfaßt. Wird für eine rasterlose Aufnahme eine Röntgenfilmkassette in das Fach eingeschoben und die Auflageplatte so gedreht, daß das Sekundärstrahlenraster in Strahlenrichtung hinter dieser Röntgenfilmkassette liegt, so kann die Belichtungszeit auch in diesem Fall durch den Belichtungsautomaten geschaltet werden. Muß in diesem Fall immer eine bestimmte Seite des Strahlendetektors von der einfallenden Röntgenstrahlung getroffen werden, so kann der Strahlendetektor um eine Achse drehbar sein, die parallel zu der Drehachse der Auflageplatte verläuft oder mit dieser zusammenfällt, so daß die jeweils erforderliche Detektorseite so nach oben gedreht werden kann, daß sie von der einfallenden Röntgenstrahlung getroffen wird.

Die Erfindung ist nachfolgend anhand der Zeichnung näher erläutert. Es zeigen:
Fig. 1 eine schematische Darstellung eines Röntgendiagnostikgerätes nach der Erfindung,
Fig. 2 und 3 die Auflageplatte des Röntgendiagnostikgerätes gemäß Figur 1 in zwei verschiedenen Stellungen, und
Fig. 4 und 5 zwei verschiedene Anordnungen des Strahlendetektors eines Röntgenbelichtungsautomaten in der Auflageplatte gemäß den Figuren 2 und 3.

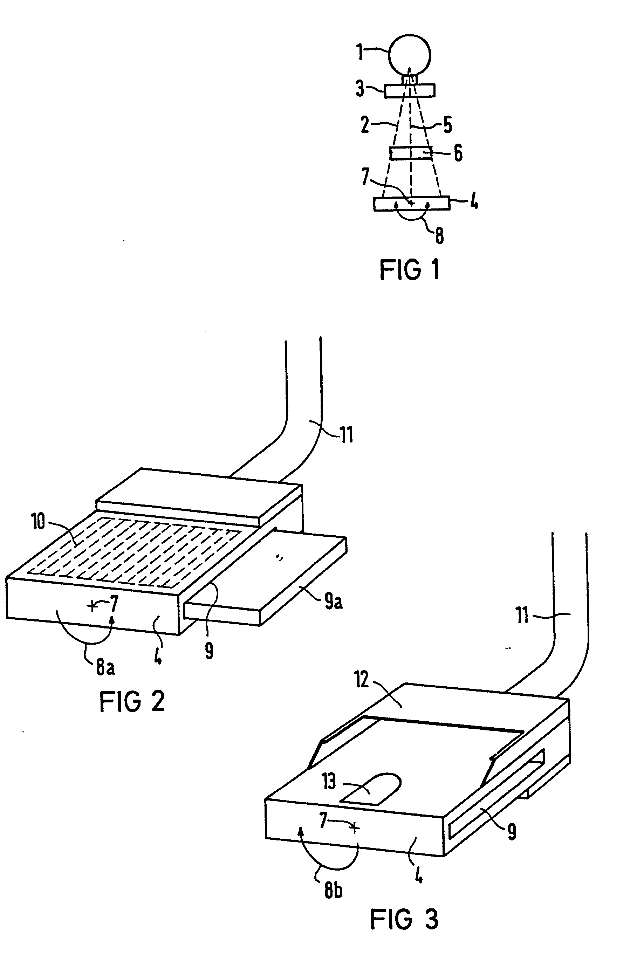

In der Figur 1 ist eine Röntgenröhre 1 dargestellt, deren Röntgenstrahlenbündel 2 aus einer Primärstrahlenblende 3 austritt. Das Aufnahmeobjekt, nämlich die Brust der zu untersuchenden Patientin, wird auf einer Auflageplatte 4 gelagert und von einer in Richtung des Zentralstrahles 5 des Röntgenstrahlenbündels 2 verstellbaren Kompressionsvorrichtung 6 komprimiert. Die Auflageplatte 4 ist um eine senkrecht zum Zentralstrahl 5 liegende Achse 7 in Richtung des Doppelpfeiles 8 drehbar gelagert, derart, daß ein in ihr angeordnetes Sekundärstrahlenraster wahlweise in Strahlenrichtung vor oder hinter der jeweils eingelegten Röntgenfilmkassette angeordnet werden kann.

Die Figur 2 zeigt, daß die Auflageplatte 4 einen seitlichen Schlitz 9 aufweist, durch den eine Röntgenfilmkassette 9a in einen Aufnahmeraum eingeschoben werden kann. In der in der Figur 2 dargestellten Stellung der Aufnahmeplatte 4 ist in Strahlenrichtung gesehen vor diesem Aufnahmeraum ein Sekundärstrahlenraster 10 angeordnet, dessen Lamellen auf den Fokus der Röntgenröhre 1 ausgerichtet sind und demgemäß die vom Aufnahmeobjekt ausgehende Streustrahlung unterdrücken. Die Auflegeplatte 4 ist an einem abgewinkelten Arm 11 um die Achse 7 drehbar gelagert und kann durch Drehung in Richtung des Pfeiles 8a in die Stellung gemäß Figur 3 verdreht werden, in der das Sekundärstrahlenraster 10 in Strahlenrichtung gesehen nach dem Aufnahmeraum für die Röntgenfilmkassette liegt. In diesem Fall, d.h. für die Anfertigung einer Röntgenaufnahme ohne Raster, wird die Röntgenfilmkassette in ein Fach 12 eingeschoben, das in der Stellung der Auflageplatte 4 gemäß Figur 2 in Strahlenrichtung gesehen unter der Röntgenfilmkassette liegt. Es ist demgemäß ein minimaler Objekt-Filmabstand bei jeder Aufnahmetechnik gewährlerstet.

Die Figur 3 zeigt schematisch noch den Strahlendetektor 13 eines Röntgenbelichtungsautomaten, der in der Stellung gemäß Figur 2 nach der Röntgenfilmkassette liegt und die Belichtungszeit automatisch optimal festlegt.

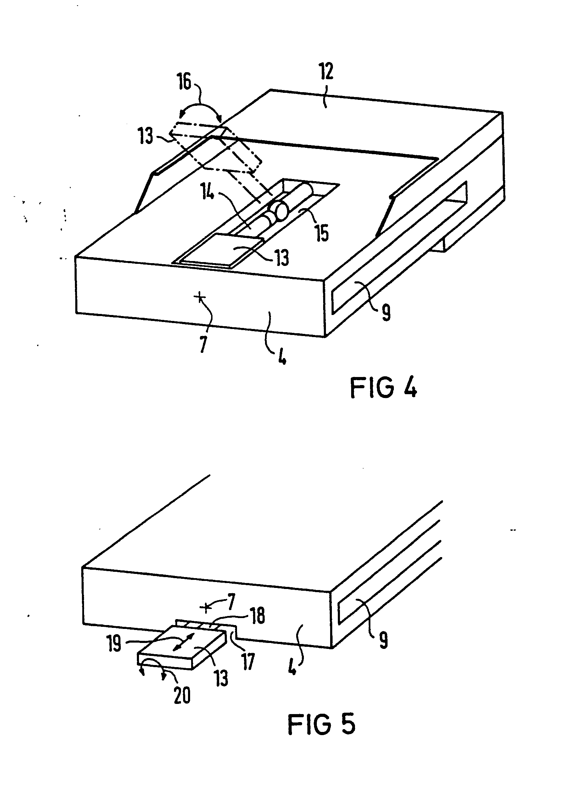

> Da diejenige Fläche eines Röntgenstrahlendetektors, auf der die Röntgenstrahlung auftrifft, normalerweise festgelegt ist, ist der Röntgenstrahlendetektor 13 gemäß Figur 4 an einem Arm 14 befestigt, der in einer Aussparung der Auflageplatte 4 drehbar gelagert ist. Auf diese Weise kann der Strahlendetektor 13 aus der Aussparung 15 herausgeklappt und in Richtung des Pfeiles 16 so gedreht werden, daß die jeweils erforderliche Seite oben liegt, d.h. der Röntgenstrahlung ausgesetzt ist.

Die Figur 5 zeigt eine Anordnung des Strahlendetektors 13, bei der dieser in einer Aussparung 17 der Auflageplatte 4 liegt, aus der er nach vorne herausgezogen werden kann oder durch Verschieben der Auflageplatte 4 nach hinten vorne heraustritt. Nun wird die Auflageplatte 4 um den Strahlendetektor 13 gedreht. Durch die Drehung wandert der Strahlendetektor 13 in eine andere Stellung (etwas höher). Die Strahleneintrittsseite bleibt jedoch der Röntgenröhre 1 zugewandt. Die Auflageplatte 4 wird durch eine Feder wieder nach vorne gedrückt oder der Strahlendetektor 13 wird wieder eingeschoben.

Die Figur 4 zeigt dabei die Stellung der Auflageplatte 4 für die Anfertigung einer rasterlosen Aufnahme gemäß Figur 3 und die Figur 5 für die Anfertigung einer Aufnahme mit Raster gemäß Figur 2. Die Auflageplatte 4 kann zur Anfertigung einer Rasteraufnahme in Richtung des Pfeiles 8b in Figur 3 wieder zurückgedreht werden.

Die Verschiebung des Strahlendetektors 13 erfolgt in der Figur 5 in Richtung des Doppelpfeiles 19 und seine Drehung in Richtung des Doppelpfeiles 20.

In der Figur 2 ist eine Röntgenfilmkassette 9a dargestellt, die in den Schlitz 9 des Aufnahmeraumes eingeschoben wird. Eine Röntgenfilmkassette gleicher Art oder größer, z. B. 24 x 30 cm kann auch in das Fach 12 eingelegt werden.

Es ist möglich, die Aufnahmeplatte so zu bemessen, daß eine im Fach 12 eingeschobene Kassette etwas über den vorderen Rand der Aufnahmeplatte 4 vorsteht. Auf diese Weise stört die Aufnahmeplatte 4 das Heranbringen der Kassette an das Aufnahmeobjekt nicht, d.h. die Kassette kann mit ihrem Rand unmittelbar an die Brustwand der Patientin angelegt werden, so daß das Aufnahmeobjekt vollständig auf dem Röntgenfilm abgebildet wird.

### Patentansprüche

1. Röntgendiagnostikgerät für Röntgenaufnahmen mit einer Auflageplatte (4) für das Aufnahmeobjekt, die einen Aufnahmeraum (9) für eine Röntgenfilmkassette (9a) und ein diesen Aufnahmeraum (9) überdeckendes Sekundärstrahlenraster (10) aufweist, dadurch gekennzeichnet, daß die Auflageplatte (4) um eine senkrecht zum Zentralstrahl (5) des Röntgenstrahlenbundels (2) liegende Achse (7) drehbar gelagert ist, derart, daß das Sekundärstrahlenraster (10) wahlweise in Strahlenrichtung vor oder hinter der jeweils eingelegten Röntgenfilmkassette (9a) angeordnet werden kann.

2. Röntgendiagnostikgerät nach Anspruch 1, dadurch gekennzeichnet, daß auf der dem Sekundärstrahlenraster (10) abgewandten Seite der Aufnahmeplatte (4) ein Fach (12) für die Aufnahme einer Röntgenfilmkassette (9a) angeordnet ist.

3. Röntgendiagnostikgerät nach Anspruch 2, bei dem auf der dem Sekundärstrahlenraster (10) abgewandten Seite der Aufnahmeplatte (4) der Strahlendetektor (13) eines Belichtungsautomaten angeordnet ist, dadurch gekennzeichnet, daß der Strahlendetektor (13) um eine Achse (14, 18) drehbar ist, die parallel zu der Drehachse (7) der Auflageplatte (4) verläuft oder mit dieser zusammenfällt.

### Claims

1. An X-ray diagnostic apparatus for X-ray photography comprising a support (4) for the object to be supported which includes a chamber (9) for an X-ray film cassette (9a) and a secondary radiation grid (10) which overlies said chamber (9), characterised in that the support (4) is mounted so as to be rotatable about an axis (7) at right-angles to the central ray (5) of the X-ray beam (2), in such a manner that the secondary radiation grid (10) can be arranged in the radiation direction either to precede or to follow the inserted X-ray film cassette (9a).

2. An X-ray diagnostic apparatus as claimed in claim 1, characterised in that a compartment (12) which accommodates an X-ray film cassette (9a) is provided on that side of the support (4) opposite the secondary radiation grid (10).

3. An X-ray diagnostic appatatus as claimed in claim 2, where the radiation detector (13) of an automatic exposure timer is arranged on that side of the support (4) opposite the secondary radiation grid (10), characterised in that the radiation detector (13) is rotatable about an axis (14, 18) which extends parallel to the rotation axis (7) of the support (4) or coincides therewith.

### Revendications

1. Appareil de radiodiagnostic pour la prise de radiographies, comportant un plateau de soutien (4) pour l'objet de prise de vue, qui comporte un espace de logement (9) pour une cassette (9a) à film radiographique et une grille antidiffusante (10) recouvrant ce logement de réception (9), caractérisé par le fait que la plaque de soutien (4) est montée de façon à pouvoir pivoter autour d'un axe (7) perpendiculaire au rayon central (5) du faisceau (2) de rayons X, de telle sorte que la grille antidiffusante (10) peut être disposée, au choix en avant ou en arrière, dans la direction du rayonnement, de la cassette (9a) à film radiographique, respectivement insérée.

2. Appareil de radiodiagnostic suivant la revendication 1, caractérisée par le fait qu'un casier (12) servant à loger une cassette (9a) à film radiographigue est disposée sur la face de la plaque de soutien (4), tournée à l'opposé de la grille antidiffusante (10).

3. Appareil de radiodiagnostic suivant la revendication 2, dans lequel le détecteur de rayonnement (13) d'un dispositif automatique d'exposition est disposé sur la face du plateau de soutien (4), située à l'opposé de la grille antidiffusante (10), caractérisé par le fait que le détecteur de rayonnement (13) peut tourner autour d'un axe (14, 18), qui est parallèle à l'axe de rotation (7) du plateau de soutien (4) ou coïncide avec oet axe.

FIG 1

FIG 2

FIG 3

16

12

13

14

15

13

7

4

9

**FIG 4**

7

18

19

17

13

4

9

20

**FIG 5**